# EUROPEAN PATENT APPLICATION

(11) **EP 2 436 317 A2**
(43) Date of publication of application: **04.04.2012**
(21) Application number: 11250787.6
(22) Date of filing: 09.09.2011
(51) Int. Cl.: A61B 17/06

(54) **Connectable end effector**

(30) Priority: 01.10.2010 US 896084
(71) Applicant: Tyco Healthcare Group LP, New Haven, CT 06511 (US)
(72) Inventor: Okoniewski, Gregory, North Haven, CT 06473 (US); Belcheva, Nadya, Hamden, CT 06518 (US)
(74) Representative: Soames, Candida Jane

(57) **Abstract**

Sutures (400) are provided having an elongate body (410) and a separately connectable end effector (420). The elongate body (410) includes a first connection structure (440), which connects the end effector (420) to the elongate body (410).

## Description

### BACKGROUND

### Technical Field

The present disclosure describes sutures including end effectors, and more particularly, sutures having connection structures which are connectable to a separate end effector.

### Background of Related Art

Medical sutures may be formed from a variety of materials and may be configured for use in limitless applications. The proximal end of the suture may have a sharpened tip, or may include a needle, for penetrating tissue. The user may frequently tie a knot at a distal end of the suture to maintaining the suture in engagement with the tissue as the suture is pulled through the tissue. Alternatively, the distal end of the suture may include an anchor or end effector for maintaining the suture in engagement with the tissue as the suture is pulled through the tissue. In other words, the end effector and the suture are provided to the user as one unitary structure. In certain situations, it may be preferable for the user to choose a different size or shaped end effector.

### SUMMARY

Sutures are described herein which comprise an elongate body including a distal portion having a first connection structure; and a separate end effector selectively connectable to the first connection structure.

In certain embodiments, the end effector comprises an extension projecting therefrom, the extension being selectively connectable to the first connection structure.

The extension may further comprise tabs or projections and the first connection structure comprises slots shaped to receive the tabs or projections. The tabs or projections may lock into the slots upon insertion of the extension into the elongate body, preventing removal of the end effector.

The end effector is shaped to prevent the end effector from being pulled through tissue, and in embodiments, the end effector has an outer diameter outer diameter which is greater than an outer diameter of the elongate body. The end effector may include a loop or a knot. In some embodiments, the end effector may comprise a second connection structure.

First or second connection structures may be selected from the group consisting of a ball connection, a socket connection, a threaded connection, and a flared connection. First or second connection structures may form a snap fit connection.

Sutures of the present disclosure include a first connection structure which is shaped to receive the end effector, for example, the first connection structure may comprise a cavity. In some embodiments, the end effector matingly cooperates with the first connection structure. The first connection structure may also be releasably connected to the end effector.

Additionally, sutures described herein may comprise a needle disposed at a proximal portion of the elongate body. In other embodiments, sutures may include projections disposed on an outer surface thereof.

Further, it may be advantageous for a suture manufacturer to produce an end effector which is separate from the suture itself. This would enable more combinations of sutures and end effectors which could be connected together.

In general, it would be beneficial to provide a suture which includes a connection structure, to enable various end effectors to be interchangeably connected with the suture.

### BRIEF DESCRIPTION OF THE DRAWING

These and other characteristics of the present invention will be more fully understood by reference to the following detailed description in conjunction with the attached drawings, in which:
FIG. 1 is a side view of a knotted end effector in accordance with one embodiment of the present disclosure;
FIG. 2 is a side view of a looped end effector in accordance with another embodiment of the present disclosure;
FIGS. 3A-3M illustrate various end effectors in accordance with other embodiments of the present disclosure;
FIGS. 4A-4C are perspective views of one embodiment of a suture in accordance with the present disclosure;
FIG 5 is a perspective view of another embodiment of a suture in accordance with the present disclosure;
FIG 6A-6I illustrate various connection structures in accordance with the present disclosure;
FIGS. 7A-7C are perspective, top and side views, respectively, of another embodiment of a suture in accordance with the present disclosure;
FIG. 8 is another embodiment of a second connection member in accordance with the present disclosure; and,
FIG. 9 is a cross-sectional view of a separate embodiment of a suture in accordance with the present disclosure.

### DETAILED DESCRIPTION

The present disclosure describes sutures including an elongate body having first connection structure, and a separate end effector which is selectively connectable to the first connection structure. In some embodiments, the separate end effector may include a second connection structure.

The term "suture" as used herein is broadly defined as a medical device which may be used to approximate tissues during wound healing. Sutures described herein may include both a connectable end effector and a needle disposed at one end of the elongate body. Sutures of the present disclosure may be provided with materials comprising both absorbable and non-absorbable materials. As used herein, the term "absorbable" includes both biodegradable and bioresorbable materials. By biodegradable, it is meant that the materials decompose, or lose structural integrity under body conditions (e.g., enzymatic degradation, hydrolysis) or are broken down (physically or chemically) under physiologic conditions in the body (e.g., dissolution) such that the degradation products are excretable or absorbable by the body.

Suitable absorbable materials include those selected from the group consisting of polymers selected from the group consisting of aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide)bioabsorbable polymers; and copolymers, block copolymers, homopolymers, blends, and combinations thereof.

More specifically, for the purpose of this invention, aliphatic polyesters include, but are not limited to, homopolymers and copolymers of lactide (including lactic acid, D-,L- and meso lactide); glycolide (including glycolic acid); epsilon-caprolactone, p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof. In certain embodiments, the mesh may comprise an aliphatic polyester.

Other suitable biodegradable polymers include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Suitable non-absorbable materials which may be employed in the present disclosure include those such as polyolefins such as polyethylene (including ultra high molecular weight polyethylene) and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers and copolymers, such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins, such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof.

In certain embodiments, both absorbable and non-absorbable materials may be employed in the suture. For example, a non-absorbable elongate body may be provided with an absorbable end effector. Alternatively, an absorbable elongate body may be provided with a non-absorbable end effector.

It should be noted that sutures of the present disclosure include both monofilament and multifilament sutures. Further, sutures of the present disclosure may include a needle attached at one end thereof. Suitable needles include those within the purview of those skilled in the art.

Methods for forming sutures in accordance with the present disclosure include techniques within the purview of those skilled in the art, such as, for example, extrusion, molding and/or solvent casting. In some embodiments, suture may include a yarn made of more than one filament, which may contain multiple filaments of the same or different materials. Where suture is made of multiple filaments, suture may be made using any known technique such as, for example, braiding, weaving or knitting. Suture may also be combined to produce a non-woven suture. Suture may be drawn, oriented, crinkled, twisted, commingled or air entangled to form yarns as part of the suture forming process. In one embodiment, a multifilament suture may be produced by braiding. The braiding may be done by any method within the purview of those skilled in the art.

Further, sutures described herein may be of any suitable cross-sectional shape, for example, elliptical, square, star shaped, octagonal, rectangular, polygonal and flat.

In certain embodiments, sutures described herein may comprise barbed sutures. Suitable barbed sutures include those described in US. Patent Publication Nos. 2009/0210006 and 2009/0248070 both assigned to Tyco Healthcare Group LP (North Haven, CT) the entire contents of which are incorporated by reference herein. In general, barbed sutures include barbs which may be arranged on a first portion of a length of the medical device body (suture) to allow movement of a first end of the medical device through tissue in one direction, while resisting movement in the opposite direction.

The barbs can be arranged in any suitable pattern, for example, helical, spiral, linear, or randomly spaced. The pattern may be symmetrical or asymmetrical. The number, configuration, spacing and surface area of the barbs can vary depending upon the tissue in which the suture is used, as well as the composition and geometry of the material utilized to form the suture. Additionally, the proportions of the barbs may remain relatively constant while the overall length of the barbs and the spacing of the barbs may be determined by the tissue being connected. For example, if the suture is to be used to connect the edges of a wound in skin or tendon, the barbs may be made relatively short and more rigid to facilitate entry into this rather firm tissue. Alternatively, if the suture is intended for use in fatty tissue, which is relatively soft, the barbs may be made longer and spaced further apart to increase the ability of the suture to grip the soft tissue.

The surface area of the barbs can also vary. For example, fuller-tipped barbs can be made of varying sizes designed for specific surgical applications. For joining fat and relatively soft tissues, larger barbs may be desired, whereas smaller barbs may be more suitable for collagen-dense tissues. In some embodiments, a combination of large and small barbs within the same structure may be beneficial, for example when a suture is used in tissue repair with differing layer structures. In particular embodiments, a barbed suture may have both large and small barbs.

As previously mentioned, sutures of the present disclosure include a separately connectable end effector. End effectors provide resistance to help prevent the suture from being pulled through tissue. Often the end effector may be used in place of a surgeon tying a knot at one end of the suture line. End effectors may be larger in cross-sectional diameter (compared to the elongate body) so that suture pull through is prevented. In other embodiments, end effectors are sized and shaped so as to mitigate suture pull through. Examples of suitable end effectors include a knotted end effector such as one described in U.S. Publication No. 2010/0094337, filed on October 1, 2009, the entire contents of which are incorporated by reference herein and described hereinbelow.

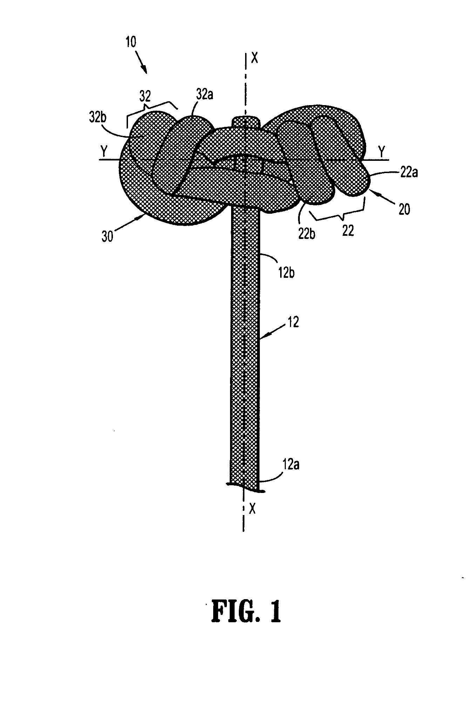

Figure 1 illustrates one embodiment of an end effector 10, which is configured to prevent complete reception of suture 12 through tissue or other material. End effector 10 forms a substantially T-shaped knot formed on distal end 12b of suture 12. End effector 10 defines an axis "Y" extending perpendicular to a longitudinal axis "X" of suture 12. End effector 10 includes first and second extensions 20, 30 extending perpendicularly from suture 12 in opposite directions along axis "Y" to form a T-shape. Each of the first and second extension 20, 30 is formed from a plurality of throws 22a-b, 32a-b, respectively, thereby forming undulated members. As used herein, a throw is defined as an at least three-hundred and sixty degree (360°) wrapping or weaving of two limbs resulting in an undulated, wavelike or rippled appearance. As shown, first and second extensions 20, 30 each include two throws 22a-b, 32a-b, respectively. It is envisioned, however, that first and second extensions 20, 30 may include any number of throws 22, 32, respectively. It is further envisioned that the number of throws on first extension 20 does not need to be equal to the number of throws on second extension 30. A proximal end 12a of suture 12 may include one or more needles (not shown) and/or may include one or more barbs.

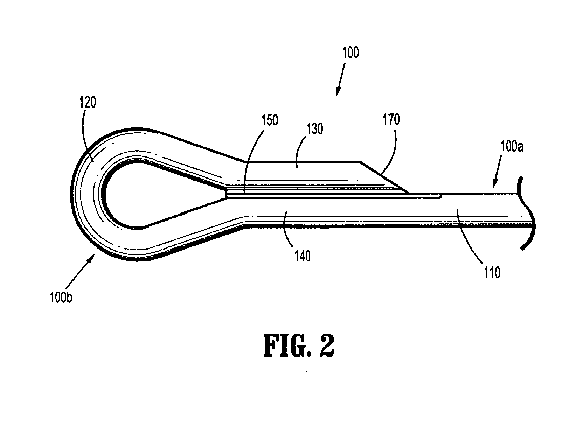

Figure 2 illustrates another exemplary end effector including a loop having first and second overlapping sections; the first overlapping section includes a surface which is tapered with respect to the longitudinal axis. The looped end effector, described in more detail below, is also described in U.S. Publication No. 2010/0063540, filed on August 29th, 2009, the entire contents of which are incorporated by reference herein.

Turning back to Figure 2, the looped suture 100 including a loop 120 formed on a distal end 100b thereof. Proximal end 100a of looped suture 100 may include one or more suture needles (not shown). Loop 120 forms a substantially teardrop shape and may be of any size. In one embodiment, loop 120 is sized to receive proximal end 100a of looped suture 100. A first section 130 of monofilament thread 110 overlays a second section 140 of thread 110 to form loop 120. The adjacent surfaces of first and second sections 130, 140 form a joined segment or joint 150.

In one embodiment, first and second sections 130, 140 of thread 110 are welded together as disclosed in U.S. Provisional Application No. 61/099,594, filed Sep. 24, 2008, the entire content of which is incorporated by reference herein. In this manner, first and second sections 130, 140 of thread 110 are locally heated until each fuses to form weld segment 150. Various types of energy may be used to locally heat first and second sections 130, 140 to form joined segment 150, including, radio frequency (RF), ultrasonic, laser, electrical arc discharge, and thermal. Alternatively, first and second sections 130, 140 of thread 110 may be joined using glues, epoxies, solvents, or other adhesives.

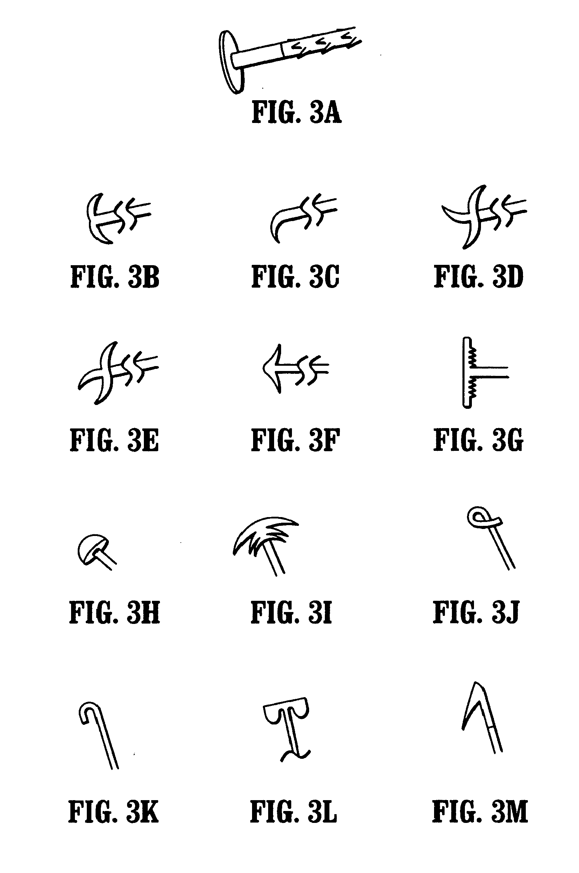

Other suitable end effectors which may be connectable to the elongate body in accordance with the present disclosure include those illustrated in Figures 3A-3M. It should be understood that end effectors in accordance with the present disclosure are not limited to those described herein and other separate end effectors may be utilized in accordance with the present disclosure.

As previously mentioned, end effectors described herein are provided as a separate structure, which is then connectable to the elongate body. In some embodiments, the elongate body includes a first connection structure, which is selectively connectable to the end effector. Connection structures in accordance with the present disclosure are discussed herein below and may include a ball connection, a socket connection, a snap-fit connection, a threaded connection, and a flared connection.

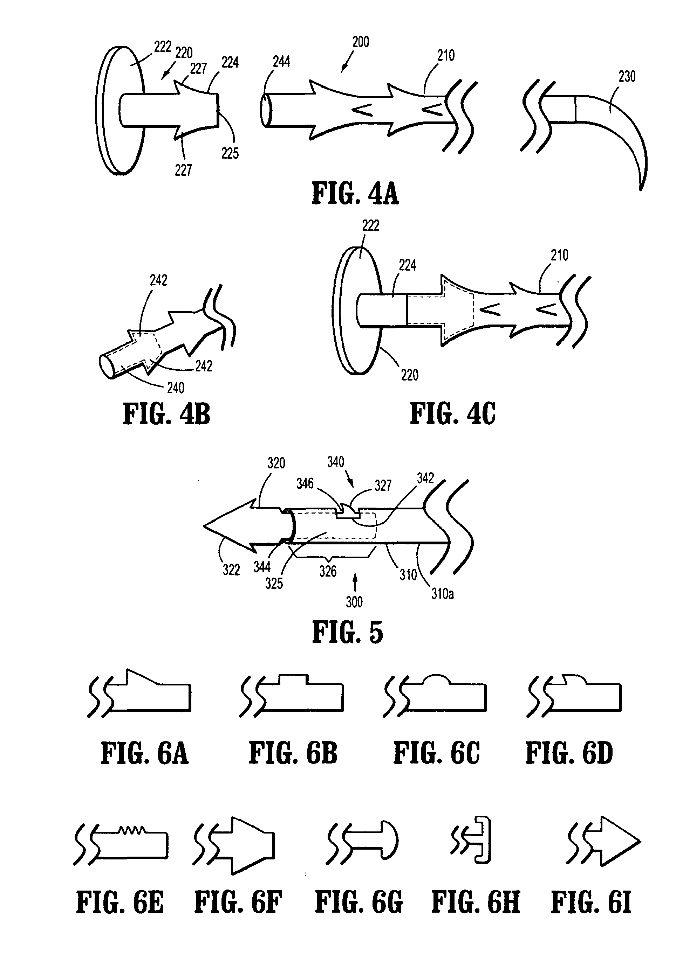

One example of a suture in accordance with the present disclosure is illustrated in Figures 4A-4C. The suture 200 includes an elongate body 210 and a separately connectable end effector 220. A proximal end of the elongate body includes a needle 230 and a distal portion of the elongate body 210 includes a first connection structure 240. As shown herein, the first connection structure 240 includes a cavity 244 comprising at least two pockets 242. The first connection structure 240 matingly cooperates with the end effector 220.

More specifically, the end effector 220 includes a distal portion 222 and a proximal portion 224. The distal portion 222 of the end effector 220 is bulb-shaped to mitigate suture pull through. Further, the distal portion of the end effector has an outer diameter which is greater than the outer diameter of the elongate body. The proximal portion 224 includes an extension 225 and at least two tabs 227. The extension 225 is shaped to be received within the distal portion of the elongate body 210. More specifically, the tabs 227 are shaped to be received within the pockets 242 (Figure 4C). In some embodiments, the pockets 242 and the tabs 227 form an irreversible connection. Once connected, the configuration of the pockets 242 and tabs 227 prevent the end effector 220 from being removed or disconnected from the elongate body 210.

Figure 5 illustrates another example of a suture 300 having a barbed elongate body 310 and a separately connectable end effector 320, in accordance with the present disclosure. A proximal end of the elongate body may be configured for needle attachment. Various needles for use are within the purview of those skilled in the art. The distal portion of the elongate body 310 includes a first connection structure 340. Similar to Figure 4A, the first connection structure 340 includes a cavity 344 which enables insertion of an extension 325. Additionally, the first connection structure 340 also includes slot 346, extending from the outer surface of the elongate body 310a to the cavity 344 of the elongate body. The slot 346 is sized and shaped for reception of a projection 327 from the surface of the end effector 320.

More specifically, the end effector 320 includes a distal portion and a proximal portion. The distal portion of the end effector 320 is arrow-shaped 322 to mitigate suture pull through. The proximal portion includes an extension 325 and at least one projection 327 formed on a surface thereof. The extension 325 is shaped to be received within the distal portion of the elongate body 310. More specifically, the projection 327 is shaped to be received within the slots 346 (on distal portion of the elongate body). In some embodiments, the slots 346 and the projections 327 form a snap fit or a friction fit. Once connected, the snap fit feature of the slots 346 and projections 327 prevent the end effector 320 from being removed or disconnected from the elongate body 310.

The projections may be a variety of shapes, including but not limited to those illustrated in Figures 6A-6H. The projections may extend from at least one surface of the end effector such as those illustrated in Figures 6A-6E. Similarly, projections or a series of projections 6A-6H may extend from the surface of the end effector. For example, figure 6A is illustrated as extending from a first surface of the suture, while Figures 6F -6I illustrate projections extending from a first and second surface of the end effector.

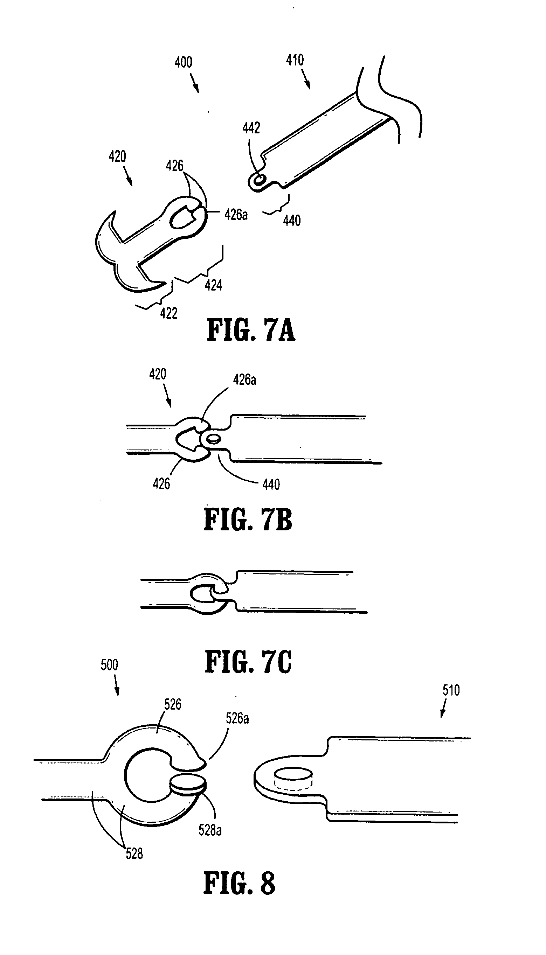

Figures 7A- 7C illustrate an alternate embodiment of a suture in accordance with the present disclosure. More particularly, the suture 400 includes an elongate body 410 and a separately connectable end effector 420. A proximal end of the elongate body includes may include a needle (not shown) and a distal portion of the elongate body 410 includes a first connection structure 440. The first connection structure 440 includes a hole or perforation 442, which is shaped and sized to receive a second connection structure.

More specifically, the end effector 420 includes a distal portion 422 and a proximal portion 424. The second connection structure comprises two flexible arms 426 which slightly separate as the arms 426 are moved into communication with the hole 442 (FIG. 7B). The arms 426 each comprise a bulbous portion 426a which slides across the first connection structure until the hole is reached. Once the bulbous portions 426a reach the hole 442, the bulbous portions 426a slide into the hole 442. Once the end effector and the elongate body are connected, the arms return to their original, unflexed position (FIG. 7C).

The first connection structure 440 can also be used with a different second connection structure, such as, for example, one illustrated in Figure 8. The second connection structure 500 shown in Figure 8 includes two arms 526, 528, respectively; the first arm 526 terminates in a bulbous portion 526a. The second arm 528 terminates in a recess configuration 528a which is shaped and sized to receive the bulbous portion 526a. The second connection structure 500 functions in a similar manner as the second connection structure 426 (Figures 7A-7C). In some embodiments, the arms are flexible and slightly separate as they are moved into communication with the hole on a first connection structure (not shown). Once the two arms 526, 528 reach the hole, the first arm 526 is received within the hole and connects to the second arm 528. More specifically, the bulbous portion 526a is received within the recess configuration 528a. The two arms 526 and 528 may require a small manual force to be connected together. As shown, the first and second arms, 526 and 528, create a snap fit connection. In embodiments, the snap fit connection may be reversible.

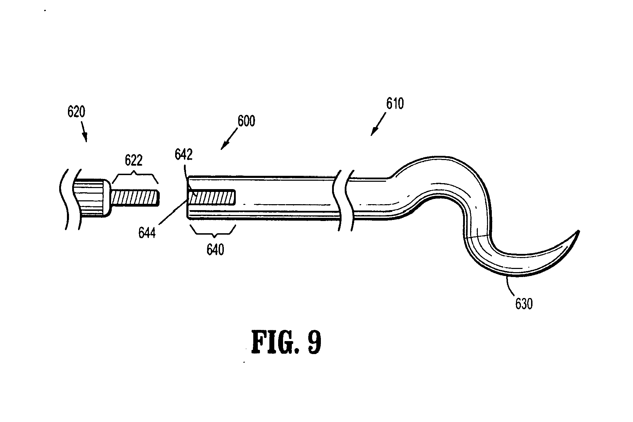

An alternative embodiment of first and second connection structures are illustrated in Figure 9. The suture 600 includes an elongate body 610 and a separately connectable end effector 620. A proximal end of the elongate body includes a needle 630 and a distal portion of the elongate body 610 includes a first connection structure 640. As shown herein, the first connection structure 640 has an interior concentric threaded portion 642. The concentric threaded portion 642 surrounds a cavity 644 in the distal portion of the elongate body 610. The cavity 644 enables insertion of a second connection structure 622 into the elongate body 610.

Similar to other embodiments, the end effector 620 includes a distal portion (not shown) which is shaped to mitigate suture pull through. A proximal portion of the end effector includes a second connection structure 622 which comprises a threaded extension 622. The threaded extension 622 is shaped to be received within first connection structure 640 and more particularly, within the cavity 644. The threaded extension 622 made comprise a right-handed or left-handed thread, corresponding to the threaded portion 642 of the first connection structure. The extension 622 is threaded within the first connection structure 640, creating a threaded connection which is reversible. In other embodiments, however, the threaded connection may be configured to be irreversible.

The end effectors described herein may be connected to the elongate bodies during manufacturing or even in the operating room. For example, by connecting the end effectors to the elongate bodies during manufacturing, several combinations of sutures can be created and packaged. Another alternative is to have the surgeon choose the specific end effector for the patient or procedure in the operating room. For example, a suture kit may be provided having several elongate bodies and several separate end effectors. The surgeon may create a suture by connecting the separate end effector and elongate body together, utilizing the connection structures provided.

Sutures may additionally include coatings for improved performance/handling characteristics, suitable coatings are within the purview of those skilled in the art.

In certain embodiments, sutures described herein may include at least one therapeutic agent. The term "therapeutic agent," as used herein, is used in its broadest sense and includes any substance or mixture of substances that provides a beneficial, therapeutic, pharmacological, and/or prophylactic effect. The agent may be a drug which provides a pharmacological effect.

The term "drug" is meant to include any agent capable of rendering a therapeutic effect, such as, anti-adhesives, antimicrobials, analgesics, antipyretics, anesthetics (e.g. local and systemic), antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes. It is also intended that combinations of agents may be used.

Other therapeutic agents, which may be included as a drug include: anti-fertility agents; parasympathomimetic agents; psychotherapeutic agents; tranquilizers; decongestants; sedative hypnotics; sulfonamides; sympathomimetic agents; vaccines; vitamins; antimalarials; antimigraine agents; anti-parkinson agents such as L-dopa; anti-spasmodics; anticholinergic agents (e.g., oxybutynin); antitussives; bronchodilators; cardiovascular agents, such as coronary vasodilators and nitroglycerin; alkaloids; analgesics; narcotics such as codeine, dihydrocodeinone, meperidine, morphine and the like; non-narcotics, such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents, such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; and immunological agents.

Other examples of suitable agents, which may be included in the sutures described herein include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and γ-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF-B; protein inhibitors; protein antagonists; protein agonists; nucleic acids such as antisense molecules, DNA, RNA, and RNAi; oligonucleotides; polynucleotides; and ribozymes.

Some specific non-limiting examples of water-soluble drugs that may be used in the present disclosure include, lidocaine, bupivicaine, tetracaine, procaine, dibucaine, sirolimus, taxol, chlorhexidine, polyhexamethylene, thiamylal sodium, thiopental sodium, ketamine, flurazepam, amobarbital sodium, phenobarbital, bromovalerylurea, chloral hydrate, phenytoin, ethotoin, trimethadione, primidone, ethosuximide, carbamazepine, valproate, acetaminophen, phenacetin, aspirin, sodium salicylate, aminopyrine, antipyrine, sulpyrine, mepirizole, tiaramide, perixazole, diclofenac, anfenac, buprenorphine, butorphanol, eptazocine, dimenhydrinate, difenidol, dl-isoprenaline, chlorpromazine, levomepromazine, thioridazine, fluphenazine, thiothixene, flupenthixol, floropipamide, moperone, carpipramine, clocapramine, imipramine, desipramine, maprotiline, chlordiazepoxide, clorazepate, meprobamate, hydroxyzine, saflazine, ethyl aminobenzoate, chlorphenesin carbamate, methocarbamol, acetylcholine, neostigmine, atropine, scopolamine, papaverine, biperiden, trihexyphenidyl, amantadine, piroheptine, profenamine, levodopa, mazaticol, diphenhydramine, carbinoxamine, chlorpheniramine, clemastine, aminophylline, choline, theophylline, caffeine, sodium benzoate, isoproterenol, dopamine, dobutamine, propranolol, alprenolol, bupranolol, timolol, metoprolol, procainamide, quinidine, ajmaline, verapamil, aprindine, hydrochlorothiazide, acetazolamide, isosorbide, ethacrynic acid, captopril, enalapril, delapril, alacepril, hydralazine, hexamethonium, clonidine, bunitrolol, guanethidine, bethanidine, phenylephrine, methoxamine, diltiazem, nicorandil, nicametate, nicotinic-alcohol tartrate, tolazoline, nicardipine, ifenprodil, piperidinocarbamate, cinepazide, thiapride, dimorpholamine, levallorphan, naloxone, hydrocortisone, dexamethasone, prednisolone, norethisterone, clomiphene, tetracycline, methyl salicylate, isothipendyl, crotamiton, salicylic acid, nystatin, econazole, cloconazole, vitamin B₁ , cycothiamine, vitamin B₂, vitamin B₃, vitamin B₅, vitamin B₆, vitamin B₇, vitamin B₉, vitamin B₁₂, vitamin C, nicotinic acid, folic acid, nicotinamide, calcium pantothenate, pantothenol, panthetin, biotin, ascorbic acid, tranexamic acid, ethamsylate, protamine, colchicine, allopurinol, tolazamide, glymidine, glybuzole, metoformin, buformin, orotic acid, azathioprine, lactulose, nitrogen mustard, cyclophophamide, thio-TEPA, nimustine, thioinosine, fluorouracil, tegafur, vinblastine, vincristine, vindesine, mitomycin C, daunorubicin, aclarubicin, procarbazine, cisplatin, methotrexate, benzylpenicillin, amoxicillin, penicillin, oxycillin, methicillin, carbenicillin, ampicillin, cefalexin, cefazolin, erythromycin, kitasamycin, chloramphenicol, thiamphenicol, minocycline, lincomycin, clindamycin, streptomycin, kanamycin, fradiomycin, gentamycin, spectinomycin, neomycin, vanomycin, tetracycline, ciprofloxacin, sulfanilic acid, cycloserine, sulfisomidine, isoniazid, ethambutol, acyclovir, gancyclovir, vidabarine, azidothymidine, dideoxyinosine, dideoxycytosine, morphine, codeine, oxycodone, hydrocodone, cocaine, pethidine, fentanyl, polymeric forms of any of the above drugs and any combinations thereof.

Therapeutic agents may be combined with sutures in various forms. For example, therapeutic agents may be combined with the sutures in the form of a coating. Suitable methods for coating sutures are within the purview of those skilled in the art and include, but are not limited to, spray coating, dip coating, extrusion, coextrusion, overmolding, and the like. Additionally, the therapeutic agent may be combined with polar and non-polar solvents creating a suture coating.

Therapeutic agent may also be polymerized off the surface of the suture or compounded within the polymer resin used to create the suture. In other embodiments, polymer drugs (e.g., polydiflunisol, polyaspirin, and protein therapeutics) may be utilized to create sutures of the present disclosure.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

The invention may be described by reference to the following numbered paragraphs:
1. A suture comprising an elongate body including a distal portion having a first connection structure; and a separate end effector selectively connectable to the first connection structure.
2. The suture according to paragraph 2, wherein the end effector comprises an extension projecting therefrom, the extension being selectively connectable to the first connection structure.
3. The suture according to paragraph 2, wherein the extension is disposed at a proximal portion of the end effector.
4. The suture according to paragraph 2, wherein the extension comprises tabs or projections and the first connection structure comprises slots shaped to receive the tabs or projections.
5. The suture according to paragraph 4, wherein the tabs or projections lock into the slots upon insertion of the extension into the elongate body.
6. The suture according to paragraph 4, wherein upon locking of the tabs or projections and the slots, removal of the end effector is prevented.
7. The suture according to paragraph 1, wherein the end effector is shaped to prevent the end effector from being pulled through tissue.
8. The suture according to paragraph 1, wherein the end effector has an outer diameter which is greater than an outer diameter of the elongate body.
9. The suture according to paragraph 1, wherein the end effector is selected from the group consisting of a loop and a knot.
10. The suture according to paragraph 1, wherein end effector matingly cooperates with the first connection structure.
11. The suture according to paragraph 1, wherein the first connection structure is shaped to receive the end effector.
12. The suture according to paragraph 1, wherein the first connection structure is releasably connected to the end effector.
13. The suture according to paragraph 1, further comprising a needle disposed at a proximal portion of the elongate body.
14. The suture according to paragraph 1, further comprising projections disposed on an outer surface thereof.
15. The suture according to paragraph 1, wherein the end effector comprises a second connection structure.
16. The suture according to paragraph 15, wherein the first or second connection structure is selected from the group consisting of a ball connection, a socket connection, a threaded connection, and a flared connection.
17. The suture according to paragraph 15, wherein the first and second connection structures form a snap fit connection.
18. The suture according to paragraph 1, wherein the first connection structure comprises a cavity.

## Claims

1. A suture comprising:
an elongate body including a distal portion having a first connection structure; and
a separate end effector selectively connectable to the first connection structure.

2. The suture according to claim 1, wherein the end effector comprises an extension projecting therefrom, the extension being selectively connectable to the first connection structure.

3. The suture according to claim 2, wherein the extension is disposed at a proximal portion of the end effector.

4. The suture according to claim 2 or claim 3, wherein the extension comprises tabs or projections and the first connection structure comprises slots shaped to receive the tabs or projections.

5. The suture according to claim 4, wherein the tabs or projections lock into the slots upon insertion of the extension into the elongate body, preferably wherein upon locking of the tabs or projections and the slots, removal of the end effector is prevented.

6. The suture according to any preceding claim, wherein the end effector is shaped to prevent the end effector from being pulled through tissue.

7. The suture according to any preceding claim, wherein the end effector has an outer diameter which is greater than an outer diameter of the elongate body, preferably wherein the end effector is selected from the group consisting of a loop and a knot.

8. The suture according to any preceding claim, wherein end effector matingly cooperates with the first connection structure.

9. The suture according to any preceding claim, wherein the first connection structure is shaped to receive the end effector.

10. The suture according to any preceding claim, wherein the first connection structure is releasably connected to the end effector.

11. The suture according to any preceding claim, further comprising a needle disposed at a proximal portion of the elongate body.

12. The suture according to any preceding claim, further comprising projections disposed on an outer surface thereof.

13. The suture according to any preceding claim, wherein the end effector comprises a second connection structure.

14. The suture according to claim 13, wherein the first or second connection structure is selected from the group consisting of a ball connection, a socket connection, a threaded connection, and a flared connection.

15. The suture according to claim 13, wherein the first and second connection structures form a snap fit connection.

16. The suture according to any preceding claim, wherein the first connection structure comprises a cavity.
